# EUROPÄISCHE PATENTSCHRIFT

(11) **EP 0 583 436 B1**
(45) Veröffentlichungstag und Bekanntmachung des Hinweises auf die Patenterteilung: **29.11.1995**
(21) Anmeldenummer: 93902158.0
(22) Anmeldetag: 09.01.1993
(51) Int. Cl.: A61K 7/13, A61K 7/48

(54) **VERWENDUNG EINER ZUBEREITUNG ZUR VERHINDERUNG VON HAUTANFÄRBUNGEN BEIM FÄRBEN DER HAARE, NEUE HAUTSCHUTZMITTEL SOWIE VERFAHREN ZUM FÄRBEN DER HAARE**
USE OF A PREPARATION TO PREVENT SKIN COLORATION WHEN DYEING HAIR, NEW SKIN PROTECTING AGENTS AND HAIR DYEING PROCESS
UTILISATION D'UNE PREPARATION DESTINEE A EMPECHER DES COLORATIONS DE LA PEAU LORS DE LA TEINTURE DES CHEVEUX, NOUVEAUX AGENTS DE PROTECTION DE LA PEAU ET PROCEDE DE TEINTURE DES CHEVEUX

(30) Priorität: 28.02.1992 DE 4206236
(43) Veröffentlichungstag der Anmeldung: 23.02.1994
(73) Patentinhaber: Wella Aktiengesellschaft, 64295 Darmstadt (DE)
(72) Erfinder: KISCHKA, Karl-Heinz, Dr., D-64293 Darmstadt (DE); FURUKAWA, Toshimasa, Narita-shi, Chiba (JP); MAEZAWA, Akiyo, Setagaya-ku, Tokyo (JP)
(86) Internationale Anmeldenummer: EP9300029
(87) Internationale Veröffentlichungsnummer: WO9316678

(56) Entgegenhaltungen:
- EP-A- 0 301 197
- FR-A- 1 322 480
- FR-A- 1 571 293
- US-A- 2 449 070

## Beschreibung

Gegenstand der vorliegenden Erfindung ist die Verwendung einer Zubereitung zur Verhinderung von Hautanfärbungen beim Färben der Haare, neue Hautschutzmittel sowie ein Verfahren zum Färben der Haare unter Verwendung dieser Hautschutzmittel oder der Zubereitung.

Beim Färben der Haare sind, auch bei sorgfältigster Arbeitsweise beim Auftragen des Haarfärbemittels, Hautanfärbungen nicht immer zu vermeiden. Insbesondere an den direkt an den Haaransatz angrenzenden sichtbaren Hautpartien wirken diese Hautanfärbungen unschön und störend.

Es sind zahlreiche sogenannte Farbfleckenentferner bekannt, die nach Abschluß der Haarfärbebehandlung zur Entfernung der Farbflecken eingesetzt werden können. Unabhängig von dem verwendeten Mittel, ist eine nachträgliche Entfernung von Farbflecken durch die in den Mitteln enthaltenen reinigenden Substanzen und die für die Entfernung der Farbflecken in der Regel notwendige mechanische Reibung immer eine Strapaze für die Haut. Zudem sind diese Farbfleckenentferner häufig nicht in der Lage einmal entstandene Hautanfärbungen restlos zu beseitigen.

Um der Entstehung von Hautanfärbungen beim Färben der Haare vorzubeugen, wurden daher Hautcremes in Form von Öl-in-Wasser- oder Wasser-in-Öl-Emulsionen sowie Vaseline vor Beginn der Färbebehandlung auf die direkt an den Haaransatz angrenzenden Hautpartien aufgetragen.

Es zeigte sich jedoch, daß bei Verwendung von emulsionsförmigen Hautschutzmitteln, insbesondere bei Haarfärbebehandlungen mit dunklen, farbstarken Nuancen, die Haut nicht ausreichend gegen Hautanfärbungen geschützt wird und die Nachbehandlung mit einem Farbfleckenentferner erforderlich ist.

Vaseline ist als Schutz gegen Hautanfärbungen beim Färben der Haare ungeeignet, da sie aufgrund ihrer Unlöslichkeit in Wasser nur sehr schwer, häufig nur mit Hilfe organischer Lösungsmittel, wie zum Beispiel Aceton oder Benzin, wieder von den an den Haaransatz angrenzenden Hautpartien zu entfernen ist.

Es bestand daher die Aufgabe, eine Zubereitung zur Verwendung für die Verhinderung von Hautanfärbungen beim Färben oder Tönen der Haare zur Verfügung zu stellen, die sich leicht auftragen läßt und die an den Haaransatz angrenzende Hautpartien sicher vor Hautanfärbungen beim Färben oder Tönen der Haare schützt. Die Zubereitung soll sich nach der Färbebehandlung oder nach der Tönung mit Wasser leicht und vollständig wieder entfernen lassen und zudem eine gute Hautverträglichkeit besitzen.

Es wurde nun überraschenderweise gefunden, daß die Verwendung einer Zubereitung, welche dadurch gekennzeichnet ist, daß sie eine Kombination von
(A) 27 bis 50 Gewichtsprozent Polyethylenglykol mit einem mittleren Molekulargewicht von 2700 bis 7500,
(B) 7 bis 14 Gewichtsprozent hydriertes Rizinusöl, welches mit 40 bis 60 Mol Ethylenoxid oxethyliert ist,
(C) 28 bis 42 Gewichtsprozent Glycerin und/oder Ethylhexandiol und/oder Polyethylenglykol mit einem Molekulargewicht von 100 bis 300 und
(D) 0 bis 18 Gewichtsprozent Wasser
enthält, zum Schutz der Haut vor Hautanfärbungen beim Färben oder Tönen von Haaren, die gestellte Aufgabe in hervorragender Weise erfüllt.

Ein Haarwachs, das die Zusammensetzung der erfindungsgemäß verwendeten Zubereitung aufweisen kann, ist aus der EP-A 0 301 197 bekannt. Die EP-A 0 301 197 offenbart jedoch ausschließlich die Verwendung des Haarwachses zur Frisurengestaltung.

Gegenstand der vorliegenden Erfindung ist zudem ein Hautschutzmittel, welches dadurch gekennzeichnet ist, daß es eine Kombination von
(A) 25 bis 40 Gewichtsprozent Polyethylenglykol mit einem mittleren Molekulargewicht von 2700 bis 4800,
(B) 2 bis 10 Gewichtsprozent Polyethylenglykol mit einem mittleren Molekulargewicht von 5600 bis 9000,
(C) 7 bis 14 Gewichtsprozent hydriertes Rizinusöl, welches mit 40 bis 60 Mol Ethylendioxid oxethyliert ist,
(D) 28 bis 42 Gewichtsprozent Glycerin und/oder Ethylhexandiol und/oder Polyethylenglykol mit einem Molekulargewicht von 100 bis 300 und
(E) 0 bis 18 Gewichtsprozent Wasser
enthält.

Das erfindungsgemäße Hautschutzmittel gewährleistet aufgrund seiner hochviskosen, wachsartigen, klebenden Konsistenz eine hervorragende Schutzwirkung gegen Hautanfärbungen bei der Haarfärbebehandlung der Haare, insbesondere auch beim Färben der Haare mit dunklen Farbnuancen. Das Hautschutzmittel läßt sich vor der Haarfärbebehandlung mühelos gleichmäßig auftragen, ist sehr gut hautverträglich und läßt sich nach der Haarfärbebehandlung mit Wasser leicht und vollständig wieder entfernen.

In einer besonders bevorzugten Ausführungsform enthält das erfindungsgemäße Hautschutzmittel
(A) 28 bis 32 Gewichtsprozent Polyethylenglykol mit einem mittleren Molekulargewicht von 2700 bis 4800,
(B) 2 bis 7 Gewichtsprozent Polyethylenglykol mit einem mittleren Molekulargewicht von 5600 bis 9000,
(C) 8 bis 13 Gewichtsprozent hydriertes Rizinusöl, welches mit 40 bis 60 Mol Ethylenoxid oxethyliert ist,
(D) 33 bis 38 Gewichtsprozent Glycerin und/oder Ethylhexandiol und/oder Polyethylenglykol mit einem Molekulargewicht von 100 bis 300 und
(E) 5 bis 14 Gewichtsprozent Wasser.

Zusätzlich zu den vorstehend aufgeführten Komponenten (A) bis (E) können sowohl die verwendete Zubereitung als auch das erfindungsgemäße Hautschutzmittel für kosmetische Mittel übliche und bekannte Zusatzstoffe wie parfümöle in einer Menge von 0,1 bis 2 Gewichtsprozent; Konservierungsmittel, beispielsweise Benzoesäure, Mandelsäure, Salicylsäure, Sorbinsäure, Formaldehyd, p-Hydroxybenzoesäureester, Mischungen aus Methylchlorisothiazolinon und Methylisothiazolinon oder 5-Chlor-2-(2,4-dichlorphenoxy)-phenol, in einer Menge von 0,1 bis 1 Gewichtsprozent; hautpflegende Stoffe, wie beispielsweise Allantoin, α-Bisabolol, Glycyrrhetinsäure, Myristylactat oder Cetyllactat, in einer Menge von 0,1 bis 2 Gewichtsprozent; konsistenzregulierende Substanzen, beispielsweise Montmorillonit oder Alkalimontmorillonit, in einer Menge von 0,1 bis 8 Gewichtsprozent, sowie filmbildende Substanzen, beispielsweise Chitosanlactat, in einer Menge von 0,1 bis 1 Gewichtsprozent, enthalten.

Die vorliegende Erfindung betrifft weiterhin ein Verfahren zum Färben der Haare, bei dem man auf die an den Haaransatz angrenzenden Hautpartien, üblicherweise 1 bis 2 cm breit, eine ausreichende Menge, im allgemeinen 5 bis 10 Gramm, der vorstehend genannten Zubereitung oder eines erfindungsgemäßen Hautschutzmittels, gegebenenfalls unter Verwendung eines Spachtels, gleichmäßig aufgeträgt und sodann das Haar mit einem Haarfärbemittel behandelt. Anschließend wird das gefärbte Haar mit Wasser ausgespült, gegebenenfalls mit einem Shampoo gewaschen und mit einem Haarpflegemittel, das mit Wasser wieder ausgespült wird, behandelt.

Die zuvor aufgetragene Zubereitung beziehungsweise das erfindungsgemäße Hautschutzmittel wird beim Waschen und Spülen gleichzeitig entfernt.

Die nachstehenden Beispiele sollen den Gegenstand der vorliegenden Erfindung näher erläutern.

### Beispiele

### Beispiel 1: Hautschutzmittel

30,00 g Polyethylenglykol mit einem mittleren Molekulargewicht von 2700 bis 4800
5,00 g Polyethylenglykol mit einem mittleren Molekulargewicht von 5600 bis 9000
10,00 g hydriertes Rizinusöl, welches mit 40 bis 60 Mol Ethylenoxid oxethyliert ist
35,26 g Glycerin
4,65 g Natrium-Montmorillonit
1,00 g Myristyllactat
0,01 g Chitosanlactat
0,20 g p-Hydroxybenzoesäuremethylester
0,10 g Salicylsäure
0,30 g Parfümöl
13,48 g Wasser
1̅0̅0̅,̅0̅0̅ g̅

### Beispiel 2: Hautschutzmittel

30,00 g Polyethylenglykol mit einem mittleren Molekulargewicht von 2700 bis 4800
5,00 g Polyethylenglykol mit einem mittleren Molekulargewicht von 5600 bis 9000
10,00 g hydriertes Rizinusöl, welches mit 40 bis 60 Mol Ethylenoxid oxethyliert ist
35,26 g Glycerin 4,65 g Natrium-Montmorillonit
1,00 g Myristyllactat
0,01 g Chitosanlactat
0,20 g p-Hydroxybenzoesäuremethylester
0,10 g Sorbinsäure
0,50 g α-Bisabolol
0,30 g Parfümöl
10,58 g Wasser
1̅0̅0̅,̅0̅0̅ g̅

### Beispiel 3: Hautschutzmittel

30,00 g Polyethylenglykol mit mittleren einem Molekulargewicht von 2700 bis 4800
5,00 g Polyethylenglykol mit einem mittleren Molekulargewicht von 5600 bis 9000
10,00 g hydriertes Rizinusöl, welches mit 40 bis 60 Mol Ethylenoxid oxethyliert ist
35,26 g Ethylhexandiol
4,65 g Natrium-Montmorillonit
1,00 g Myristyllactat
0,01 g Chitosanlactat
0,20 g p-Hydroxybenzoesäuremethylester
0,10 g Salicylsäure
0,30 g Parfümöl
13,48 g Wasser
1̅0̅0̅,̅0̅0̅ g̅

### Beispiel 4: Hautschutzmittel

30,00 g Polyethylenglykol mit einem mittleren Molekulargewicht von 2700 bis 4800
5,00 g Polyethylenglykol mit einem mittleren Molekulargewicht von 5600 bis 9000
12,40 g hydriertes Rizinusöl, welches mit 40 bis 60 Mol Ethylenoxid oxethyliert ist
35,26 g Polyethylenglykol mit einem Molekulargewicht von 100 bis 300
4,65 g Natrium-Montmorillonit
1,00 g Myristyllactat
0,01 g Chitosanlactat
0,20 g p-Hydroxybenzoemethylester
0,10 g Sorbinsäure
0,50 g α-Bisabolol
0,30 g Parfümöl
10,58 g Wasser
1̅0̅0̅,̅0̅0̅ g̅
Die Herstellung der Hautschutzmittel gemäß den Beispielen 1 bis 4 erfolgt, indem die Komponenten bei 65 Grad Celsius geschmolzen und miteinander vermischt werden. Die homogene Schmelze des Hautschutzmittels wird anschließend unter Rühren abgekühlt und bei 45 bis 55 Grad Celsius in Tiegel abgefüllt.

### Beispiel 5: Verfahren zum Färben der Haare

Blondes Naturhaar wird zunächst mit Wasser angefeuchtet. Anschließend werden auf die an den Haaransatz angrenzenden Hautpartien 1 bis 2 cm breit 7,5 Gramm des erfindungsgemäßen Hautschutzmittels gemäß Beispiel 4 gleichmäßig aufgetragen. Sodann werden 50 g eines Haarfärbemittels der folgenden Zusammensetzung
1,00 g 4-Amino-2[bis-(2'-hydroxyethyl)-amino-methyl]-phenol
1,10 g 1-Naphthol
15,00 g Cetylalkohol
0,30 g Natriumsulfit, wasserfrei
3,50 g Laurylalkohol-diglykolethersulfat-Natriumsalz (28 %ige wäßrige Lösung)
3,00 g Ammoniak (22 %ige wäßrige Lösung)
76,10 g Wasser
1̅0̅0̅,̅0̅0̅ g̅
kurz vor dem Gebrauch mit 50 g Wasserstoffperoxidlösung (6-prozentig) vermischt. Anschließend trägt man das Gemisch auf die blonden Naturhaare auf und läßt es 30 Minuten lang bei 40° Celsius einwirken.

Danach wird das Haar mit Wasser gespült und gleichzeitig das Hautschutzmittel entfernt. Anschließend wird das Haar getrocknet. Das Haar hat eine intensive Rotfärbung erhalten. Die an den Haaransatz angrenzenden Hautpartien sind durch das Hautschutzmittel effektiv vor Anfärbungen geschützt worden.

Sämtliche in dieser Anmeldung angegebenen Prozentzahlen stellen Gewichtsprozent dar.

## Patentansprüche

1. Verwendung einer Zubereitung mit einem Gehalt an
(A) 27 bis 50 Gewichtsprozent Polyethylenglykol mit einem mittleren Molekulargewicht von 2700 bis 7500,
(B) 7 bis 14 Gewichtsprozent hydriertes Rizinusöl, welches mit 40 bis 60 Mol Ethylenoxid oxethyliert ist,
(C) 28 bis 42 Gewichtsprozent Glycerin und/oder Ethylhexandiol und/oder Polyethylenglykol mit einem Molekulargewicht von 100 bis 300 und
(D) 0 bis 18 Gewichtsprozent Wasser
zum Schutz vor Hautanfärbungen beim Färben oder Tönen der Haare.

2. Hautschutzmittel, dadurch gekennzeichnet, daß es eine Kombination aus
(A) 25 bis 40 Gewichtsprozent Polyethylenglykol mit einem mittleren Molekulargewicht von 2700 bis 4800,
(B) 2 bis 10 Gewichtsprozent Polyethylenglykol mit einem mittleren Molekulargewicht von 5600 bis 9000,
(C) 7 bis 14 Gewichtsprozent hydriertes Rizinusöl, welches mit 40 bis 60 Mol Ethylenoxid oxethyliert ist,
(D) 28 bis 42 Gewichtsprozent Glycerin und/oder Ethylhexandiol und/oder Polyethylenglykol mit einem Molekulargewicht von 100 bis 300 und
(E) 0 bis 18 Gewichtsprozent Wasser
enthält.

3. Hautschutzmittel nach Anspruch 2, dadurch gekennzeichnet, daß es eine Kombination aus
(A) 28 bis 32 Gewichtsprozent Polyethylenglykol mit einem mittleren Molekulargewicht von 2700 bis 4800,
(B) 2 bis 7 Gewichtsprozent Polyethylenglykol mit einem mittleren Molekulargewicht von 5600 bis 9000,
(C) 8 bis 13 Gewichtsprozent hydriertes Rizinusöl, welches mit 40 bis 60 Mol Ethylenoxid oxethyliert ist,
(D) 33 bis 38 Gewichtsprozent Glycerin und/oder Ethylhexandiol und/oder Polyethylenglykol mit einem Molekulargewicht von 100 bis 300 und
(E) 5 bis 14 Gewichtsprozent Wasser
enthält.

4. Verfahren zum Färben der Haare, bei dem man auf die an den Haaransatz angrenzenden Hautpartien eine ausreichende Menge einer Zubereitung oder eines Hautschutzmittels gleichmäßig aufträgt, sodann das Haar mit einem Haarfärbemittel behandelt, das Haar anschließend mit Wasser ausspült und dabei gleichzeitig das Hautschutzmittel entfernt, dadurch gekennzeichnet, daß eine Zubereitung nach Anspruch 1 oder ein Hautschutzmittel nach einem der Ansprüche 2 oder 3 verwendet wird.

## Claims

1. Use of a preparation containing:
(A) 27 to 50 weight % of polyethyleneglycol having an average molecular weight of from 2700 to 7500;
(B) 7 to 14 weight % of hydrated castor oil oxyethylated with 40 to 60 moles ethylene oxide;
(C) 28 to 42 weight % of glycerol and/or ethylhexandiol and/or polyethyleneglycol with a molecular weight of from 100 to 300; and
(D) 0 to 18 weight % of water
for protection of the skin from coloration when the hair is coloured or tinted.

2. Skin-protection agent, characterised in that it contains a combination of
(A) 25 to 40 weight % of polyethylene glycol having an average molecular weight of from 2700 to 4800;
(B) 2 to 10 weight % of polyethylene glycol having an average molecular weight of from 5600 to 9000;
(C) 7 to 14 weight % of hydrated castor oil oxyethylated with 40 to 60 moles of ethylene oxide;
(D) 28 to 42 weight % of glycerol and/or ethylhexandiol and/or polyethylene glycol with a molecular weight of from 100 to 300; and
(E) 0 to 18 weight % of water.

3. Skin-protection agent according to claim 2 characterised in that it contains a combination
(A) 28 to 32 weight % of polyethylene glycol having an average molecular weight of from 2700 to 4800;
(B) 2 to 7 weight % of polyethylene glycol having an average molecular weight of 5600 to 9000;
(C) 8 to 13 weight % of hydrated castor oil oxyethylated with 40 to 60 moles of ethylene oxide;
(D) 33 to 38 weight % of glycerol and/or ethylhexandiol and/or polyethylene glycol having a molecular weight of 100 to 300; and
(E) 5 to 14 weight % of water.

4. Process for colouring the hair, in the case of which an adequate amount of a preparation or a skin protection agent is applied evenly to the parts of the skin adjacent the hair line, the hair is then treated with a hair-colouring agent, the hair is subsequently rinsed with water and the skin protecting agent is thus removed at the same time, characterised in that a preparation according to Claim 1 or a skin-protection agent according to either of Claims 2 and 3 is used.

## Revendications

1. Utilisation d'une préparation présentant une teneur de
(A) 27 à 50% en poids d'un polyéthylène glycol d'un poids moléculaire moyen allant de 2700 à 7500,
(B) 7 à 14% en poids d'huile de ricin hydrogéné qui est oxyéthylé par 40 à 60 moles d'oxyde d'éthylène,
(C) 28 à 42% en poids de glycérine et/ou d'éthylhexanediol et/ou de polyéthylèneglycol, avec un poids moléculaire moyen allant de 100 à 300,
(D) 0 à 18% en poids d'eau, pour la protection contre la coloration de la peau lors de la teinture ou du nuançage des cheveux.

2. Agent protecteur de la peau, caractérisé en ce qu'il contient une combinaison de :
(A) 25 à 40% en poids de polyéthylèneglycol d'un poids moléculaire moyen allant de 2700 à 4800,
(B) 2 à 10% en poids de polyéthylèneglycol d'un poids moléculaire moyen allant de 5600 à 9000,
(C) 7 à 14% en poids d'huile de ricin hydrogénée qui est oxyéthylée par 40 à 60 moles d'oxyde d'éthylène,
(D) 28 à 42% en poids de glycérine et/ou d'éthylhexanediol et/ou de polyéthylèneglycol d'un poids moléculaire moyen allant de 100 à 300,
(E) 0 à 18% en poids d'eau.

3. Agent de protection de la peau, selon la révendication 2, caractérisé en ce qu'il contient une combinaison de
(A) 28 à 32% en poids de poiyéthylèneglycol d'un poids moléculaire moyen allant de 2700 à 4800,
(B) 2 à 7% en poids de polyéthylèneglycol d'un poids moleculaire moyen allant de 5600 à 9000,
(C) 8 à 13% en poids d'huile de ricin hydrogénée qui est oxyéthylée par 40 à 60 moles d'oxyde d'éthylène,
(D) 33 à 38% en poids de glycérine et/ou d'éthylhexanediol et/ou de polyéthylèneglycol d'un poids moléculaire moyen de 100 à 300 et
(E) 5 à 14% en poids d'eau.

4. Procédé de teinture des cheveux selon lequel on applique sur les parties de peau limitant la base des cheveux, une quantité suffisante d'une préparation ou d'un agent de protection de la peau d'une façon régulière, ensuite les cheveux sont traités par un agent tinctorial, les cheveux sont alors rincés à l'eau et, de ce fait, l'agent de protection de la peau est éliminé simultanément, caractérisé en ce qu'on utilise une préparation selon la revendication 1 ou un agent de protection de la peau selon une des revendications 2 ou 3.
